# DEMANDE DE BREVET EUROPEEN

(11) **EP 3 143 956 A1**
(43) Date de publication de la demande: **22.03.2017**
(21) Numéro de dépôt: 16189121.3
(22) Date de dépôt: 16.09.2016
(51) Int. Cl.: A61B 17/88, A61F 2/46, A61B 17/00

(54) **INSTRUMENT CHIRURGICAL, KIT CHIRURGICAL ET PROCEDE DE FABRICATION AFFERENTS**

(30) Priorité: 17.09.2015 FR 1558776
(71) Demandeur: In2Bones, 69130 Ecully (FR)
(72) Inventeur: BAERTICH, Christian, 31300 Toulouse (FR); VERNOIS, Joel, 90310 Picquigny (FR); RAY, Adrien, 1227 Carouge (CH); REDFERN, David Jonathan, Hove BN3 3PA (GB)
(74) Mandataire: Weber, Jean-François

(57) **Abrégé**

- L'invention concerne un instrument chirurgical (1) destiné à permettre de modifier le positionnement de corps osseux d'un patient l'un par rapport à l'autre, par exemple de son pied, ledit instrument chirurgical (1) comportant :
- un manche de préhension (5) s'étendant le long d'un premier axe longitudinal (A-A'),
- une tête de travail, comportant un corps allongé s'étendant le long d'un deuxième axe longitudinal entre une première extrémité reliée au manche de préhension (5) et une deuxième extrémité (11) libre, ledit corps présentant une surface supérieure (12) et une surface inférieure reliant lesdites première et deuxième (11) extrémités,
ledit instrument chirurgical (1) étant caractérisé en ce que la tête de travail est pourvue d'une gorge (16) axiale ménagée dans ladite surface supérieure (12).
- Instruments chirurgicaux destinés à effectuer une modification du positionnement relatif de corps osseux.

## Description

La présente invention concerne le domaine de l'instrumentation chirurgicale, et en particulier des outils chirurgicaux destinés à effectuer une modification du positionnement relatif de corps osseux d'un patient, notamment dans le cadre du traitement d'une pathologie du pied, et à être insérés à cet effet entre les corps osseux concernés.

La présente invention concerne plus particulièrement un instrument chirurgical destiné à être manoeuvré à la manière d'un levier pour permettre de modifier le positionnement de corps osseux d'un patient l'un par rapport à l'autre, par exemple de son pied, ledit instrument chirurgical comportant :
- un manche de préhension s'étendant le long d'un premier axe longitudinal A-A',
- une tête de travail, comportant un corps allongé s'étendant le long d'un deuxième axe longitudinal B-B' entre une première extrémité reliée au manche de préhension et une deuxième extrémité libre, ledit corps présentant une surface supérieure et une surface inférieure reliant lesdites première et deuxième extrémités.

La présente invention concerne également un procédé de fabrication d'un tel instrument chirurgical.

La présente invention concerne en outre un kit chirurgical destiné à la mise en oeuvre d'une technique opératoire, préférentiellement percutanée, notamment dans le cadre du traitement d'une pathologie du pied d'un patient, comprenant un tel instrument ch irurg ical.

Dans ce cadre du traitement de certaines pathologies osseuses, notamment du pied d'un patient, il est parfois nécessaire au chirurgien de venir écarter et repositionner des corps osseux l'un par rapport à l'autre en vue de permettre leur ostéosynthèse dans une configuration anatomique préférentielle donnée.

En particulier, dans le cas du traitement d'une pathologie telle que l'*hallux valgus,* pathologie relativement courante caractérisée par une déformation du pied correspondant à la déviation du premier métatarsien en *varus* et du gros orteil (*hallux*) en *valgus,* une technique opératoire bien connue consiste à venir pratiquer une ostéotomie de la tête (métaphyse distale) du premier métatarsien, c'est-à-dire à sectionner chirurgicalement le premier métatarsien, afin d'en réorienter, manuellement ou à l'aide d'un instrument chirurgical spécifique, la tête et sa surface articulaire et de corriger ainsi la déformation. Une broche est parfois introduite axialement dans le corps (diaphyse) du métatarsien afin de maintenir les segments osseux en position corrigée le temps de leur ostéosynthèse.

Cette technique opératoire chirurgicale est classiquement réalisée « à *ciel ouvert* », c'est-à-dire en pratiquant de larges incisions dans la peau et les tissus mous de sorte à permettre au chirurgien d'accéder facilement et avec un bon contrôle visuel à la zone à traiter. Relativement lourde et imposant une hospitalisation du patient, cette technique opératoire est progressivement remplacée par une méthode moins invasive qui permet une opération en ambulatoire : la chirurgie percutanée ou « *mini-invasive ».*

Plus complexe à maitriser pour le chirurgien et nécessitant des outils chirurgicaux spéciaux souvent onéreux, cette technique se pratique à travers la peau, généralement sous anesthésie locorégionale et contrôle radiographique, par le biais de quelques incisions d'environ 2 à 5 mm de long seulement, appelée voies d'abord, destinées à permettre l'introduction dans le corps du patient des instruments et éventuels implants chirurgicaux nécessaires à l'opération.

Dans le domaine des instruments chirurgicaux destinés à effectuer une telle correction du positionnement relatif de corps osseux d'un patient, et en particulier de segments d'un os du pied, on connait des instruments manuels de type levier, aussi appelés écarteurs, consistant en une tige se terminant par une tête plus fine et courbée de sorte à former avec la tige une forme en « Z ». Ces écarteurs sont exclusivement réalisés en matériau métallique, notamment par usinage et pliage.

Une fois le premier métatarsien sectionnée, on vient écarter les segments osseux que constituent d'une part la tête et d'autre part le corps du premier métatarsien en insérant la pointe de l'écarteur dans le canal médullaire du corps du premier métatarsien et en faisant levier avec l'écarteur en prenant appui sur la tête du premier métatarsien et sur la tête de l'os du gros orteil. Pour améliorer l'appui, certains des instruments connus présentent par ailleurs une tête pourvue d'une zone élargie et sensiblement plate.

Si de tels instruments donnent généralement satisfaction, ils restent cependant peu ergonomiques et ne sont en particulier pas adaptés à des opérations chirurgicales nécessitant la mise en place de certains instruments spécifiques, notamment d'une broche d'ostéosynthèse pour assurer la fusion des segments osseux.

En effet, la pose d'une telle broche impose au chirurgien de retirer au préalable son instrument du canal médullaire du premier segment osseux et donc de relâcher l'effort d'écartement exercé sur les corps osseux. De ce fait, le positionnement d'une broche d'ostéosynthèse se révèle relativement complexe et imprécis.

L'invention vise en conséquence à porter remède aux différents inconvénients énumérés précédemment, et à proposer un instrument chirurgical plus polyvalent et en particulier destiné à permettre de modifier le positionnement de corps osseux d'un patient l'un par rapport à l'autre, permettant la mise en oeuvre simultanée d'une broche d'ostéosynthèse de manière particulièrement précise.

Un autre objet de l'invention vise à proposer un nouvel instrument chirurgical, destiné à permettre de modifier le positionnement de corps osseux d'un patient l'un par rapport à l'autre, permettant la mise en oeuvre précise d'une broche d'ostéosynthèse, quel que soit le diamètre de celle-ci.

Un autre objet de l'invention vise à proposer un nouvel instrument chirurgical, destiné à permettre de modifier le positionnement de corps osseux d'un patient l'un par rapport à l'autre, particulièrement ergonomique et facile à manipuler.

Un autre objet de l'invention vise à proposer un nouvel instrument chirurgical, destiné à permettre de modifier le positionnement de corps osseux d'un patient l'un par rapport à l'autre, sur et relativement peu coûteux à fabriquer.

Un autre objet de l'invention vise à proposer un nouveau procédé de fabrication d'un instrument chirurgical à la fois simple et peu coûteux à mettre en oeuvre.

Un autre objet de l'invention vise à proposer un nouveau kit chirurgical destiné à la mise en oeuvre d'une technique opératoire, préférentiellement percutanée, complet, ergonomique, sur et peu coûteux.

Les objets assignés à l'invention sont atteints à l'aide d'un instrument chirurgical destiné à être manoeuvré à la manière d'un levier pour permettre de modifier le positionnement de corps osseux d'un patient l'un par rapport à l'autre, par exemple de son pied, ledit instrument chirurgical comportant :
- un manche de préhension s'étendant le long d'un premier axe longitudinal A-A',
- une tête de travail, comportant un corps allongé s'étendant le long d'un deuxième axe longitudinal B-B' entre une première extrémité reliée au manche de préhension et une deuxième extrémité libre, ledit corps présentant une surface supérieure et une surface inférieure reliant lesdites première et deuxième extrémités,
ledit instrument chirurgical étant caractérisé en ce que la tête de travail est pourvue d'une gorge axiale ménagée dans ladite surface supérieure.

Les objets assignés à l'invention sont également atteints à l'aide d'un procédé de fabrication d'un tel instrument chirurgical, caractérisé en ce que ledit procédé comprend une unique étape de moulage au cours de laquelle on réalise ledit instrument chirurgical d'un seul tenant dans son intégralité.

Les objets assignés à l'invention sont également atteints à l'aide d'un kit chirurgical destiné à la mise en oeuvre d'une technique opératoire, préférentiellement percutanée, notamment dans le cadre du traitement d'une pathologie du pied d'un patient, comprenant un tel instrument chirurgical, et étant caractérisé en ce qui comprend également au moins un manche porte-lame de bistouri, une rugine et une râpe.

D'autres objets et avantages de l'invention apparaîtront plus en détail à la lecture de la description qui suit, ainsi qu'à l'aide des dessins annexés fournis à titre purement explicatif et non limitatif, dans lesquels :
- La figure 1 illustre, en vue de profil, un instrument chirurgical conforme à l'invention;
- Les figures 2 et 3 illustrent, respectivement en perspective et en vue de face, l'instrument de la figure 1, de sorte à faire particulièrement ressortir les configurations respectives de la gorge et des ailettes dont est pourvue la tête de travail de cet instrument ;
- La figure 4 illustre, en perspective, l'instrument chirurgical des figures 1 à 3, en faisant notamment ressortir ses caractéristiques de courbure, et en particulier, les inclinaisons relatives du manche de préhension, de la tête de travail et de sa pointe de travail ;
- La figure 5 illustre, en vue de profil, un exemple de mise en oeuvre conjuguée de l'instrument chirurgical de l'invention et d'une broche d'ostéosynthèse ;
- La figure 6 illustre un exemple de mise en oeuvre conjuguée de l'instrument chirurgical de l'invention et d'une broche d'ostéosynthèse en relation avec des corps osseux dans le cas d'un traitement d'une pathologie du pied d'un patient par ostéotomie ;
- La figure 7 illustre, en vue de dessus, un mode de réalisation préférentiel d'un kit chirurgical conforme à l'invention ;
- La figure 8 illustre en vue de profil un mode de réalisation d'une râpe telle que préférentiellement incluse dans le kit chirurgical de la figure 7 ;
- Les figures 9 à 12 illustrent différentes configurations géométriques que peuvent présenter les dents de la râpe de la figure 8.

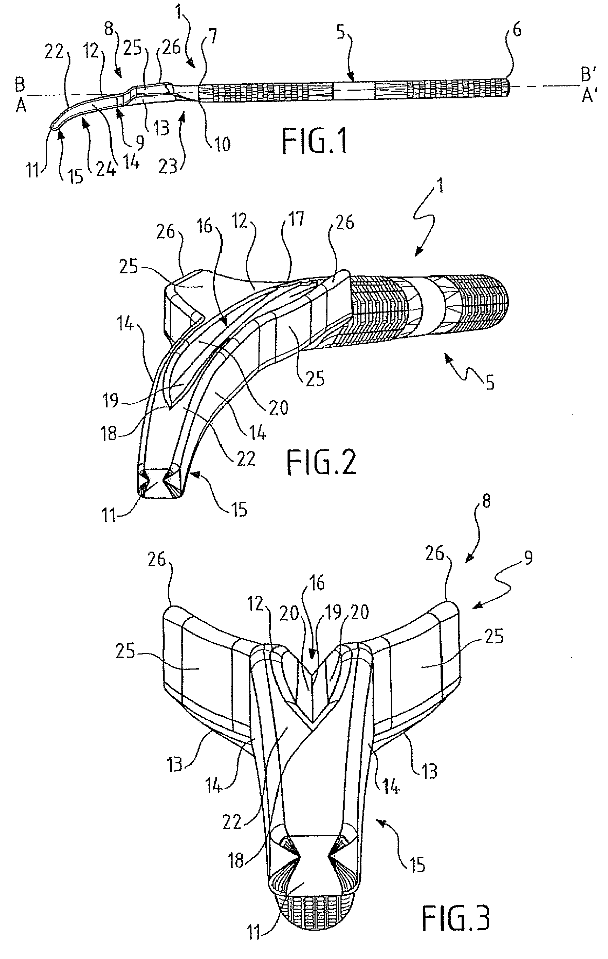

L'invention concerne un instrument chirurgical 1, avantageusement de type écarteur, destiné à être manoeuvré à la manière d'un levier pour permettre de modifier le positionnement de corps osseux 2, 3 d'un patient l'un par rapport à l'autre, par exemple de son pied, dont un exemple de réalisation conforme à l'invention est illustré aux figures 1 à 5. En l'espèce, l'instrument chirurgical 1 de l'invention est particulièrement adapté à être utilisé par un chirurgien au cours d'une opération chirurgicale percutanée sur un patient, en particulier dans le cadre du traitement d'une pathologie du pied par ostéotomie, par exemple d'un *hallux valgus.*

On entend par « *corps osseux* 2, 3 » des os, des cartilages, des fragments d'os ou de cartilage, de préférence destinés à être fusionnés par ostéosynthèse après avoir fait l'objet d'une modification de leur positionnement relatif à l'aide de l'instrument chirurgical 1. De manière encore plus préférentielle, l'invention prend tout son sens lorsque ces corps osseux 2, 3 sont au nombre de deux et sont constitués de segments séparés d'un même os, typiquement le corps d'un os doté d'un canal médullaire 4 et sa tête. On entend par ailleurs par la modification du positionnement de corps osseux 2, 3, le fait de déplacer lesdits corps osseux 2, 3, préférentiellement à l'aide de l'instrument 1 de l'invention, pour en changer l'orientation ou le positionnement relativement l'un à l'autre, et ce par exemple en vue de rectifier un alignement osseux à des fins thérapeutiques ou plastiques.

Bien entendu, sans sortir du cadre de l'invention, l'instrument chirurgical 1 pourra être utilisé dans le cadre du traitement d'une pathologie affectant d'autres os que ceux du pied, par exemple des os de la main, dès lors qu'il est nécessaire déplacer des corps osseux 2, 3 pour en modifier ou en corriger le positionnement. Non restreint à un usage exclusif à un patient humain, l'instrument chirurgical 1 pourra également être mise en oeuvre dans le cadre d'une chirurgie animale pour le traitement de pathologies osseuses similaires.

Selon l'invention, et comme illustré à la figure 1, ledit instrument chirurgical 1 comporte un manche de préhension 5, préférentiellement à préhension manuelle, s'étendant le long d'un premier axe longitudinal A-A'. L'instrument chirurgical 1 est en effet préférentiellement à préhension manuelle, c'est-à-dire qu'il peut être manoeuvré directement à la main par le chirurgien. Rien ne s'oppose toutefois à ce qu'instrument 1 puisse être mis en oeuvre d'une manière autre que manuelle, par exemple à l'aide d'un moyen robotisé. Préférentiellement de forme cylindrique, ce manche 5 s'étend avantageusement de manière longitudinale entre une extrémité proximale 6 et une extrémité distale 7.

L'instrument chirurgical 1 de l'invention comporte également une tête de travail 8, cette dernière comportant un corps 9 allongé s'étendant le long d'un deuxième axe longitudinal B-B', entre une première extrémité 10 reliée au manche de préhension 5, préférentiellement au niveau de l'extrémité distale 7 de ce dernier, et une deuxième extrémité 11 libre. Ladite tête de travail 8 s'étend avantageusement dans le prolongement du manche de préhension 5, lesdits premier A-A' et deuxième B-B' axes étant préférentiellement confondus. Il est toutefois possible d'envisager une configuration alternative, non illustrée aux figures, dans laquelle ladite tête de travail 8 ne s'étendrait pas dans le prolongement du manche de préhension 5, mais serait au contraire reliée audit manche 5 de façon déportée. En outre, lesdits premier A-A' et deuxième B-B' axes sont avantageusement compris dans un même plan.

Ladite tête de travail 8 est préférentiellement reliée en sa première extrémité 10 au manche de préhension 5 de façon ferme, ladite tête de travail 8 et ledit manche de préhension 5 étant rendu solidaires l'un de l'autre. La tête de travail 8 peut constituer une pièce indépendante du manche de préhension 5, éventuellement interchangeable, rapportée et maintenue fermement à ce dernier par exemple par collage, vissage, clipsage ou encore par enfichage. Toutefois, de manière préférentielle, ledit manche de préhension 5 et ladite tête de travail 8 constituent une pièce monobloc d'un seul tenant, c'est-à-dire qu'ils constituent avantageusement une seule et même pièce réalisée en totalité dans un matériau donné.

Ledit corps 9 présente une surface supérieure 12 et une surface inférieure 13 reliant lesdites première 10 et deuxième 11 extrémités. Ces surfaces supérieure 12 et inférieure 13 forment le contour externe de la tête de travail 8 et de son corps 9, exclusivement entre lesdites première 10 et deuxième 11 extrémités. Ces surfaces supérieure 12 et inférieure 13 n'incluent pas les première 10 et deuxième 11 extrémités.

De façon préférentielle, le corps 9 de la tête de travail 8 s'étend :
- latéralement entre deux bords latéraux 14, et
- transversalement entre lesdites surfaces supérieure 12 et inférieure 13,
lesdits bords latéraux 14 et lesdites surfaces supérieure 12 et inférieure 13 étant respectivement sensiblement parallèles sur au moins une portion de la longueur de la tête de travail 8, puis convergents vers l'extrémité libre 11 de cette dernière de sorte à former en cette extrémité libre 11 une pointe de travail 15. En d'autres termes, si le corps 9 de la tête de travail 8 présente une section rectangulaire, la pointe de travail 15 présente elle-même une section rectangulaire, mais d'aire inférieure à celle de la section de la tête de travail 8. De la sorte, et tel qu'illustré aux figures, la tête de travail 8 présente, au moins au niveau de sa pointe de travail 15, une forme effilée particulière apte, par exemple, à être insérée dans une incision étroite réalisée dans la peau et dans les tissus mous dans le cadre d'une opération de type percutané, de sorte à venir faire coopérer la pointe de travail 15 avec le canal médullaire 4 d'un corps osseux 2, par exemple d'un segment osseux de premier métatarsien.

Selon une caractéristique importante de l'invention, la tête de travail 8 est pourvue d'une gorge 16 axiale, avantageusement linéaire, c'est-à-dire s'étendant selon le deuxième axe longitudinal B-B' du corps 9 de la tête de travail 8, qui est ménagée dans ladite surface supérieure 12. Ladite gorge 16 axiale s'étend avantageusement entre une ouverture proximale 17 et une ouverture distale 18, lesdites ouvertures proximale 17 et distale 18 débouchant de la surface supérieure 12 du corps 9 de ladite tête de travail 8. Ladite gorge 16 ne constitue donc pas une rainure fermée, mais bien une sorte de gouttière ouverte en chacune de ses extrémités.De façon préférentielle, ladite gorge 16 est formée par :
- un fond de gorge 19 s'étendant, depuis ladite ouverture proximale 17, au niveau de laquelle la tête de travail 8 est reliée au manche de préhension 5, le long de l'axe longitudinal B-B' sur au moins une portion de la longueur de la tête de travail 8,
- deux parois 20 de gorge s'élevant à partir du fond 19 jusqu'à la surface supérieure 12.

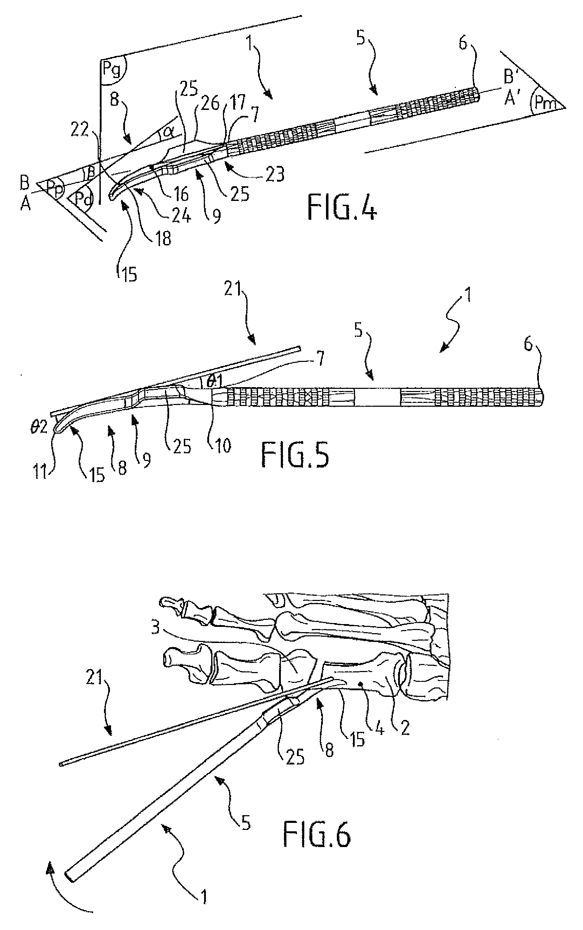

Tel qu'illustré aux figures, l'ouverture proximale 17 de la gorge 16 est préférentiellement confondue avec la première extrémité 10 du corps 9 de la tête de travail 8, de sorte que ladite gorge 16 s'étend depuis la première extrémité 10 sur une longueur plus ou moins longue de la tête de travail 8. La gorge 16 est ainsi avantageusement apte à recevoir et à guider un corps allongé et fin, telle qu'une broche 21 d'ostéosynthèse, par exemple des broches de Kirschner, celle-ci pouvant venir se loger et coulisser dans ladite gorge 16 de part et d'autre de ces ouvertures proximale 17 et distale 18, tel qu'illustré à la figure 5. La broche 21 forme alors, respectivement au niveau desdites ouvertures proximale 17 et distale 18, un angle Θ1, Θ2 avec la tête de travail. Par ailleurs, la longueur de la gorge 16 le long de l'axe longitudinal B-B' permet en pratique à l'instrument chirurgical 1 de recevoir et de guider de manière précise des broches 21 rigides et longues. Dans le cas préférentiel illustré notamment aux figures 2 et 3, ladite gorge 16 ne s'étend en direction de l'extrémité 11 libre de la tête de travail 8 cependant que jusqu'au voisinage de la base 22 de la pointe de travail 15, de manière à ne pas fragiliser mécaniquement ladite pointe de travail 15.

De manière encore plus avantageuse, et tel qu'illustré notamment aux figures 2 et 3, lesdites parois 20 sont configurées de sorte à conférer à ladite gorge 16 un profil sensiblement conique, c'est-à-dire que les parois 20 sont préférentiellement inclinées l'une par rapport à l'autre en direction du fond de gorge 19 de manière à former un profil en « *V* ». Une telle configuration est particulièrement intéressante, car elle permet de fait à la gorge 16 de recevoir et de guider tout aussi précisément des broches 21 de diamètres différents, typiquement de diamètres compris entre 1,0 et 2,5 mm, lesdites broches 21 venant en contact avec les parois 20 plus ou moins profondément dans la gorge 16.

Dans le mode de réalisation préférentiel illustré aux figures, ladite gorge 16 présente par ailleurs une forme fuselée, ses parois 20 convergeant vers l'ouverture proximale 17.

L'instrument chirurgical 1 étant destiné à être manoeuvré par le chirurgien à la manière d'un levier pour permettre le déplacement relatif de corps osseux 2, 3, la tête de travail 8 ne s'inscrit pas dans le plan d'extension moyen Pm du manche de préhension 5, dans lequel est inscrit le premier axe longitudinal A-A' d'extension dudit manche 5, mais présente au contraire une forme courbe. Toutefois, et contrairement aux instruments de type écarteurs classiques, la tête de travail 8 de l'instrument chirurgical 1 n'est pas configurée de manière à former avec le manche 5 une forme courbée en « *Z* ». En effet, te! qu'illustré aux figures 1 à 4, ladite tête de travail 8 est préférentiellement recourbée, c'est-à-dire qu'elle présente une forme générale courbe continue, sans changement de direction, le corps 9 et la pointe de travail 15 de la tête de travail 8 tendant tous deux à s'éloigner du plan d'extension moyen Pm du manche de préhension 5. Ainsi, et tel qu'illustré à la figure 4, le corps 9 de la tête de travail 8 s'étend préférentiellement sensiblement le long d'un plan d'extension proximal Pp sécant au premier axe longitudinal A-A', hormis sa pointe de travail 15 qui s'étend quant à elle avantageusement sensiblement selon un plan d'extension distal Pd sécant au plan d'extension proximal Pp. Ladite pointe de travail 15 s'étend en outre, de préférence, selon un axe longitudinal moyen compris dans le plan que le deuxième axe B-B'. Le corps 9 de la tête de travail 8 et le manche de préhension 5 sont ainsi reliés par une première zone de courbure 23, tandis que ladite pointe de travail 15 et ladite tête de travail 8 sont avantageusement reliées par une deuxième zone de courbure 24. La tête de travail 8 présente ainsi une double inflexion, la pointe de travail 15 étant fléchie relativement au reste du corps 9 de la tête de travail 8 et ledit corps 9 étant lui-même fléchi relativement au manche de préhension 5, les première 23 et deuxième 24 zones de flexion étant avantageusement respectivement localisées au niveau de la première extrémité 10 de la tête de travail 8 reliée au manche de préhension 5 et au niveau de la base 22 de la pointe de travail 15. Plus précisément, le corps 9 de la tête de travail 8 est incliné par rapport au manche de préhension 5 de sorte que le plan d'extension proximal Pp et le premier axe longitudinal A-A' sont sécants l'un par rapport à l'autre de manière à former un premier angle d'élévation α avantageusement compris entre 30° et 60°, et de façon plus préférentielle d'environ 45°, tandis que la pointe de travail 15 est inclinée par rapport au corps 9 de la tête de travail 8 de sorte que le plan d'extension distal Pd et le plan d'extension proximal Pp sont sécants l'un par rapport à l'autre de manière à former un deuxième angle d'élévation β avantageusement compris entre 30° et 60°, et de façon plus préférentielle d'environ 45°.

De manière particulièrement avantageuse, lesdites ouverture proximale 17 et ouverture distale 18 de la gorge 16 débouchant de la surface supérieure 12 du corps 9 de ladite tête de travail 8 sont respectivement positionnées au niveau desdites première 23 et deuxième 24 zones de courbure. De la sorte, tel qu'illustré à la figure 6, la combinaison d'une telle configuration recourbée de la tête de travail 8 de l'instrument 1 avec ladite gorge 16 axiale, au profil avantageusement conique, permet ainsi de déplacer deux segments ou corps osseux 2, 3 l'un par rapport à l'autre par un mouvement simple de levier et de fixer l'ostéosynthèse grâce à une broche 21 sans avoir à retirer l'instrument 1 puisque cette dernière peut librement coulisser dans ladite gorge 16 ménagée dans la surface supérieure 12 de la tête de travail 8, la fonction de levier liée à la courbure de la tête de travail 8 n'entravant pas la fonction de guidage offerte par la gorge 16. L'instrument 1 permet par conséquent, outre d'assurer sa fonction première de déplacement de segments ou corps osseux 2, 3, un positionnement simultané, aisé et plus précis, de ladite broche 21, respectueux de l'orientation choisie par le chirurgien. De façon préférentielle, et tel qu'illustré aux figures 1 à 5, la tête de travail 8 de l'instrument chirurgical 1 est dotée de deux ailes 25 longitudinales. Ces ailes 25 font saillie du corps 9 allongé de ladite tête de travail 8 de sorte à en prolonger lesdites surfaces supérieure 12 et inférieure 13, étant disposées symétriquement par rapport à un plan d'extension Pg de la gorge, préférentiellement orthogonal au plan d'extension distal Pd de la tête de travail 8, et de façon radiale à l'axe longitudinal B-B'. De manière particulièrement avantageuse, et tel qu'illustré notamment aux figures 2 et 3, lesdites ailes longitudinales 25 sont incurvées de sorte à conférer localement à ladite surface supérieure 12 une forme sensiblement concave et à ladite surface inférieure 13 une forme sensiblement convexe. En d'autres termes, les ailes 25 sont configurées relativement au corps 9 de la tête de travail 8, de sorte à ce que leurs bords longitudinaux 26, préférentiellement parallèles, s'élèvent en s'éloignant du plan d'extension distal Pd de la tête de travail 8 et, avantageusement, dans une direction opposée à celle de la courbure générale ci-avant décrite de ladite tête de travail 8. De la sorte, et en reprenant l'exemple, illustré à la figure 6, d'un traitement d'une pathologie du pied d'un patient par ostéotomie, lorsque la pointe de travail 15 de l'instrument 1 est insérée dans le canal médullaire 4 d'un premier segment ou corps osseux 2, par exemple d'un premier métatarsien, la surface supérieure 12 ainsi pourvue d'ailes 25 vient avantageusement épouser le contour d'un deuxième segment ou corps osseux 3, par exemple la tête de ce premier métatarsien, de manière à offrir un appui de large et stable à l'instrument 1 lorsque ce dernier est manoeuvré tel un levier pour modifier le positionnement de ces premier 2 et deuxième 3 corps osseux. Ainsi, l'instrument 1 est particulièrement ergonomique, pratique et sur d'utilisation, puisque le risque de glissement, ou ripage, de l'instrument 1 sur les corps osseux 2, 3, et en particulier deuxième segment ou corps osseux 3, sous l'effort de levier exercé par le chirurgien est particulièrement limité.

N'ayant pas nécessairement vocation, dans le cas d'une mise en oeuvre percutanée, à être insérées dans le corps du patient, lesdites ailes 25 longitudinales sont préférentiellement positionnées à proximité de ladite première extrémité 10 de la tête de travail 8, c'est-à-dire l'extrémité au niveau de laquelle ladite tête 8 est reliée au manche 5, et s'étendent longitudinalement sensiblement sur la moitié de la longueur de ladite tête de travail 8. Ceci permet avantageusement de conserver exempte d'ailes 25 une portion de la longueur de la tête de travail 8 étroite et suffisamment longue pour être insérée dans le corps du patient au travers d'une incision de 2 à 5 mm environ et permettre ainsi à l'instrument 1 de jouer efficacement son rôle de levier.

De façon préférentielle, l'instrument chirurgical 1 décrit ci-dessus est à usage unique, c'est-à-dire qu'il est destiné à être détruit ou recyclé / valorisé à l'issue de son utilisation pour un patient et une opération chirurgicale donnés. Cela permet non seulement une plus grande sécurité sanitaire, mais également un gain économique important pour le chirurgien et le centre de soins dans lequel il exerce, les coûts liés au nettoyage, à la stérilisation et au reconditionnement d'instruments chirurgicaux réutilisables étant particulièrement élevés en regard du coût de l'instrument en lui-même. A ce titre, ledit instrument chirurgical 1 est avantageusement réalisé en matériau polymère, par exemple un matériau polymère composite à base de polyarylamide (PAA), ce dernier étant optionnellement chargé, comme par exemple un matériau polymère renforcé à base de polyarylamide de la gamme « *IXEF* »® commercialisée par la société SOLVAY. Un tel matériau présente en effet l'avantage d'être un matériau biocompatible doté d'une bonne résistance à la fatigue et d'une bonne ténacité. Il peut optionnellement être chargé en fibres, par exemple de carbone ou de verre, de manière à conférer à l'instrument chirurgical 1 une grande rigidité et une excellente résistance mécanique en flexion. De plus, les matériaux à base de polyarylamide sont connus pour être particulièrement faciles à mettre en oeuvre, en particulier par moulage par injection, même à haute teneur en fibres. Ils présentent un faible retrait au moulage et permettent ainsi la réalisation précise et répétable de pièces de faibles dimensions et épaisseurs et de formes complexes.

L'invention concerne en outre en tant que tel un procédé de fabrication d'un instrument chirurgical 1 tel que décrit précédemment dans sa variante selon laquelle son manche de préhension 5 et sa tête de travail 8 constituent une pièce monobloc d'un seul tenant, ledit procédé comprenant une unique étape de moulage, par exemple une étape de moulage par injection, au cours de laquelle on réalise ledit instrument chirurgical 1 d'un seul tenant dans son intégralité. En d'autres termes, le procédé de fabrication de l'invention permet de réaliser ledit instrument chirurgical 1 en une pièce monobloc d'un seul tenant, en une seule opération, sans autre opération de finition du genre usinage ou autre. La matière destinée à être moulée au cours du procédé de fabrication est avantageusement un matériau polymère, préférentiellement un matériau polymère composite à base de polyarylamide (PAA) tel que mentionné ci-avant.

L'invention concerne également en tant que tel un kit chirurgical 27 destiné à la mise en oeuvre d'une technique opératoire, préférentiellement percutanée, notamment dans le cadre du traitement d'une pathologie du pied d'un patient, comprenant un instrument chirurgical 1 conforme à l'invention, et tel que décrit ci-avant, et comprenant également au moins un manche porte-lame de bistouri 28, une rugine 29 et une râpe 30.

Le manche porte-lame 28 est apte à recevoir de manière amovible une lame de bistouri (non représentée). Dans le cas pris en exemple d'un kit chirurgical 27 destiné à la réalisation d'opérations percutanées, ce manche porte-lame 28 équipé d'une lame de bistouri est destiné à permettre la réalisation de très fines incisions de la peau et des tissus mous constituant des voies d'abord à travers lesquelles seront introduits dans le corps du patient les instruments et éventuels implants chirurgicaux nécessaires à l'opération.

La rugine 29 est un outil chirurgical destiné à racler une surface osseuse et, en particulier, à séparer des os les tissus mous environnants tels que des muscles ou des tendons, afin de pouvoir travailler en contact direct de ladite surface osseuse. Une telle rugine 29 est formée d'une petite plaque supportée par un manche. Ladite plaque est pourvue d'arêtes vives et son extrémité libre, qui forme une arête perpendiculaire à l'axe principal du manche, est biseautée de manière à former un bord d'attaque spécifiquement agressif pour assurer le décollement du périoste et des tissus mous.

Ladite plaque et ledit manche forment préférentiellement une pièce monobloc d'un seul tenant. Ladite rugine 29 peut toutefois alternativement être formée d'une plaque et d'un manche distincts, ladite plaque étant insérée dans le manche de manière permanente ou encore amovible.

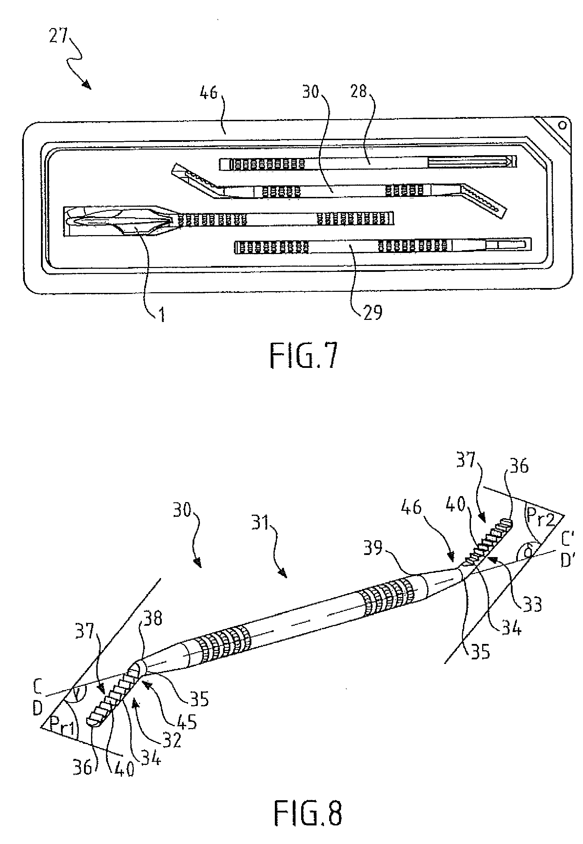

La râpe 30 (ou *« excavateur »*), dont un exemple de réalisation est illustré à la figure 8, est un outil chirurgical qui permet d'extraire du corps du patient des débris biologiques ou séquestre, constitués notamment de fragments osseux et tissulaires mélangés à du sang, en particulier ceux générés lors d'une opération d'ostéotomie, par exemple du premier métatarsien, ou de réduction du volume de la tête métatarsienne.

Une telle râpe 30 comporte préférentiellement :
- d'une part, d'un manche de râpe 31 s'étendant le long d'un troisième axe longitudinal C-C', et
- d'autre part, d'au moins une tête de râpe 32, 33, comportant un corps 34 allongé s'étendant le long d'un quatrième axe longitudinal D-D' entre une première extrémité 35 reliée au manche de râpe 31 et une deuxième extrémité 36 libre, ledit corps 34 présentant une face active 37 reliant lesdites première 35 et deuxième 36 extrémités.

Ladite au moins une tête de râpe 32, 33 s'étend avantageusement dans le prolongement du manche de râpe 31, de préférence à partir d'une des extrémités 38, 39 de ce dernier, lesdits troisième C-C' et quatrième D-D' axes étant préférentiellement confondus.

Ladite face active 37 de la tête de râpe 32, 33 est pourvue d'une rangée de dents 40 parallèles, alignées selon le quatrième axe longitudinal D-D', et présentant des arêtes 41 vives aptes à venir râper efficacement les corps osseux 2, 3 et tissus mous environnants afin d'extraire le séquestre.

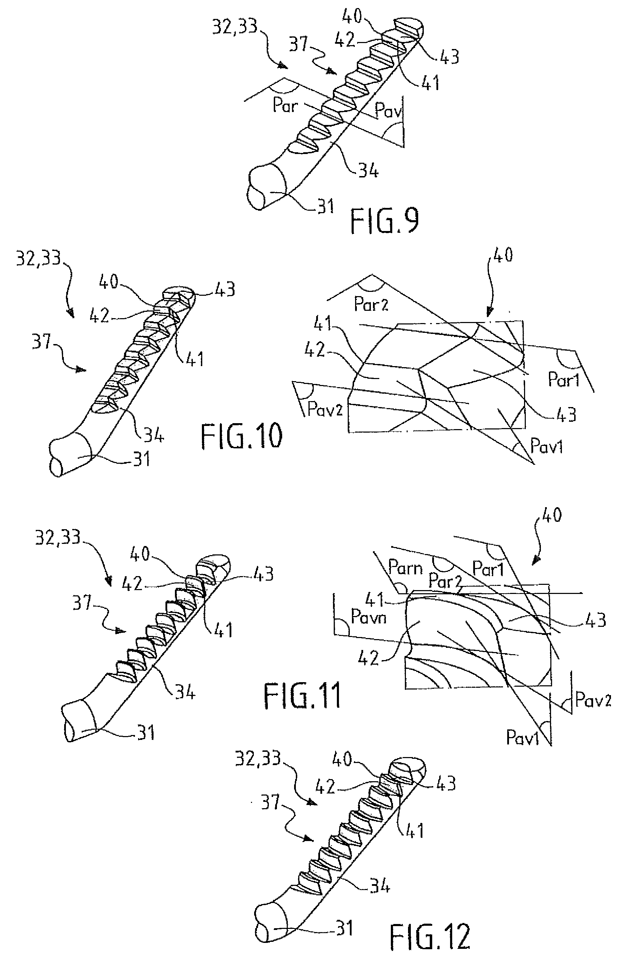

Classiquement ces dents 40 se présentent selon une configuration « *droite* », illustrée à la figure 9, c'est-à-dire qu'elles s'étendent linéairement dans une direction orthogonale au quatrième axe longitudinal D-D', leurs faces avant 42 et arrière 43 s'inscrivant respectivement dans un seul et unique plan Pav, Par.

De manière préférentielle, les faces avant 42 et arrière 43 desdites dents 40 s'inscrivent respectivement dans au moins deux plans Pav1, Pav2, Par1, Par2 sécants distincts, de sorte que lesdites dents 40 peuvent avantageusement présenter une configuration géométrique alternative parmi au moins les suivantes :
- une configuration *« en chevron » :* selon cette première variante, illustrée à la figure 10, les faces avant 42 et arrière 43 desdites dents 40 s'inscrivent respectivement dans deux plans Pav1, Pav2, Par1, Par2 sécants distincts, de sorte que lesdites dents 40 présentent un profil concave en « *V »,* l'ouverture du « V » s'ouvrant préférentiellement en direction du manche de râpe 31,
- une première configuration « *concave* » : selon cette seconde variante, illustrée à la figure 11, les faces avant 42 et arrière 43 desdites dents 40 s'inscrivent préférentiellement respectivement dans une multitude de plans Pav1, Pav2,..., Pavn, PAr1, Par2,..., Parn sécants distincts, de sorte que lesdites dents 40 présentent un profil concave curviligne, la concavité des dents 40 s'ouvrant de préférence en direction du manche de râpe 31 ;
- une deuxième configuration « *concave » :* selon cette troisième variante, illustrée à la figure 12, les faces avant 42 et arrière 43 desdites dents 40 s'inscrivent préférentiellement dans une multitude de plans Pav1, Pav2,..., Pavn, PAr1, Par2,..., Parn sécants distincts, de sorte que lesdites dents 40 présentent un profil concave curviligne, la concavité des dents 40 s'ouvrant de préférence en direction opposée à celle du manche de râpe 31.

Il a en effet été observé que ces configurations « *en chevron* » et concaves, en comparaison avec la configuration « *droite »* classique, permettent avantageusement de bloquer dans la concavité de chaque dent davantage de séquestres osseux et ainsi de les extraire de la plaie plus efficacement et rapidement, ce qui contribue à la réduction du temps opératoire.

Bien évidemment, les dents 40 de ladite tête de râpe 32, 33, peuvent présenter d'autres variantes avantageuses de configuration géométrique que celles décrites ci-dessus, par exemple une configuration selon un profil en «*W*».

Le corps 34 de ladite tête de râpe 32, 33 est préférentiellement relié en sa première extrémité 35 au manche de râpe 31 de façon ferme, ladite tête de râpe 32, 33 et ledit manche de râpe 31 étant rendu solidaires l'un de l'autre. La tête de râpe 32, 33 peut ainsi constituer une pièce indépendante du manche de râpe 31, éventuellement interchangeable, rapportée et maintenue fermement à ce dernier par exemple par collage, vissage, clipsage ou encore par enfichage.

Toutefois, de manière préférentielle, ledit manche de râpe 31 et ladite tête de râpe 32, 33 constituent une pièce monobloc d'un seul tenant, c'est-à-dire qu'ils constituent avantageusement une seule et même pièce réalisée en totalité dans un matériau donné, préférentiellement un matériau polymère, par exemple un matériau polymère composite à base de polyarylamide (PAA), ce dernier étant optionnellement chargé.

De façon préférentielle, et tel qu'illustré à la figure 8, ladite râpe 30 comporte avantageusement deux têtes de râpe 32, 33, chacune reliée à l'une des extrémités 38, 39 du manche de râpe 30.

Le corps 34 des têtes de râpe 32, 33 s'étendent respectivement sensiblement le long d'un premier Pr1 et d'un deuxième Pr2 plan d'extension sécants au troisième axe longitudinal C-C', le corps 34 desdites têtes de râpe 32, 33 et le manche de râpe 31 étant respectivement reliés par une première 45 et une deuxième 46 zone d'inflexion.

Dans une variante préférentielle illustrée à la figure 8, ladite râpe 30 comporte deux têtes de râpes 32, 33. Celles-ci s'étendent respectivement dans un plan d'extension Pr1, Pr2, lesdits plans Pr1, Pr2 étant parallèles et sécants au troisième axe longitudinal C-C' de sorte à former avec ce dernier un troisième angle élévation γ, δ préférentiellement compris entre 135° et 165°.

En outre, dans cette variante préférentielle, lesdites têtes de râpes 32, 33 s'étendent avantageusement en sens opposé, conférant à ladite râpe une forme en « Z », de sorte à ce que le chirurgien, lorsqu'il utilise manuellement l'une des têtes de râpe 32, 33, ne soit pas gêné par la présence de l'autre tête de râpe 32, 33.

D'autres variantes sont toutefois parfaitement envisageables, les plans d'extension Pr1, Pr2 pouvant ne pas être parallèles et les angles d'élévation γ, δ respectivement formés par ces plans avec le troisième axe longitudinal C-C' pouvant être identiques ou différents, tout en étant avantageusement compris entre 135° et 165°.

Pour finir, lorsque ladite râpe 30 se présente dans une variante dans laquelle elle comporte deux têtes de râpe 32, 33, les dents 40 de celles-ci peuvent présenter des configurations géométriques, telles que décrites précédemment, identiques ou bien différentes, de sorte à offrir au chirurgien une râpe 30 parfaitement adaptée à ses besoins.

Il est entendu que la râpe 30 ci-dessus décrite, prise indépendamment dudit kit chirurgical 27, peut en tant que telle constituer une invention à part entière, distincte de la présente invention.

Sans sortir du cadre de l'invention, le kit chirurgical 27 pourra également inclure d'autres outils chirurgicaux que ceux décrits ci-dessus, par exemple une ou plusieurs broches 21 d'ostéosynthèse, par exemple des broches de Kirschner, ou encore une ou plusieurs fraises percutanées permettant de réaliser des découpes osseuses.

Tel qu'illustré à la figure 7, ledit kit 27 est avantageusement constitué d'un étui 46, par exemple en plastique transparent de type polypropylène, dans lequel sont disposés lesdits instrument chirurgical 1, manche porte-lame de bistouri 28, rugine 29 et râpe 30, ces derniers étant préférentiellement calés dans ledit étui 46 par une mousse de calage.

De manière préférentielle, ledit kit chirurgical 27 est à usage unique, c'est-à-dire que lesdits instrument chirurgical 1, manche porte-lame de bistouri 28, rugine 29 et râpe 30 qui le composent sont destinés à être détruits ou recyclés / valorisés à l'issue de leur utilisation pour un patient et une opération chirurgicale donnés.

Il est cependant envisageable, sans sortir du cadre de l'invention, que ledit kit 27 soit au contraire réutilisable, c'est-dire que lesdits instrument chirurgical 1, manche porte-lame de bistouri 28, rugine 29 et râpe 30 qui le composent pourraient, après une première utilisation, être à nouveau proposés au chirurgien après avoir subi un retraitement et un reconditionnement adéquat.

A ce titre, lesdits instrument chirurgical 1, manche porte-lame de bistouri 28, rugine 29 et râpe 30 sont avantageusement sont tous réalisés en matériau polymère, par exemple un matériau polymère composite à base de polyarylamide (PAA), ce dernier étant optionnellement chargé.

De manière encore plus préférentielle, lesdits instrument chirurgical 1, manche porte-lame de bistouri 28, rugine 29 et râpe 30 sont avantageusement conditionnés de manière stérile dans l'étui 46, de sorte à être immédiatement utilisables par le chirurgien sans étape de nettoyage et de stérilisation préalable.

Enfin, l'invention pourra concerner en tant que telle une méthode d'utilisation de l'instrument chirurgical 1 selon l'invention afin de modifier le positionnement relatif de corps osseux 2, 3, par exemple dans le cadre du traitement chirurgical d'une pathologie du pied d'un patient tel qu'illustré à la figure 6.

Une telle méthode comprend avantageusement au moins les étapes successives suivantes :
- on incise le corps du patient, typiquement sur quelques millimètres (par exemple environ 2 à 5 mm) afin de pouvoir introduire au moins partiellement la tête de travail 8 de l'instrument chirurgical 1 à l'intérieur, au niveau des corps osseux 2, 3, par exemple au niveau d'un premier segment osseux 2 formé du corps d'un premier métatarsien et d'un deuxième segment osseux 3 formé de la tête de ce premier métatarsien ;
- on écarte lesdits corps osseux 2, 3 l'un par rapport à l'autre à l'aide de la tête de travail 8, pour venir insérer la pointe de travail 15 de la tête de travail 8 dans le canal médullaire 4 d'un premier corps osseux 2 ;
- on continue à écarter et déplacer lesdits corps osseux 2, 3 l'un par rapport à l'autre en prenant appui sur le second corps osseux 3, préférentiellement en venant faire coopérer la partie concave de la surface supérieure 12 dotée d'ailes longitudinales 25 avec la surface du deuxième corps osseux 3, et en exerçant un effort de levier sur le manche de préhension 5 jusqu'à obtenir le positionnement préférentiel souhaité desdits corps osseux 2, 3.

De manière préférentielle, ladite méthode comprend une étape supplémentaire, consécutive à celles décrites ci-avant, dans laquelle, tout en maintenant l'effort de levier exercé sur lesdits corps osseux 2, 3 à l'aide de l'instrument 1 afin de conserver le positionnement préférentiel obtenu, on vient faire coulisser une broche 21 d'ostéosynthèse dans la gorge 16 dudit instrument, depuis l'ouverture proximale vers l'ouverture distale de la dite gorge 16, de sorte à positionner et fixer de manière précise ladite broche 21 dans le canal médullaire dudit premier corps osseux 3.

Une telle méthode pourra bien évidemment être mise en oeuvre, par voie chirurgicale percutanée ou « *à ciel ouvert* », dans le cadre du traitement d'une pathologie affectant d'autres os que ceux du pied, par exemple des os de la main, dès lors qu'il est nécessaire déplacer des corps osseux 2, 3 pour en modifier ou en corriger le positionnement.

En définitive, l'instrument chirurgical 1 de l'invention, particulièrement ergonomique et simple d'utilisation, permet au chirurgien de modifier le positionnement de corps osseux 2, 3 d'un patient l'un par rapport à l'autre, tout en autorisant avantageusement la mise en oeuvre d'une broche 21 d'ostéosynthèse de manière particulièrement précise et sans avoir à retirer l'instrument chirurgical 1. Réalisé à partir d'un procédé de fabrication simple, l'instrument chirurgical 1 est relativement peu couteux à produire. Il est en outre avantageusement intégré dans le kit chirurgical 27 de l'invention, de manière à offrir au chirurgien un ensemble complet, ergonomique, sur et peu coûteux, d'outils destinés la mise en oeuvre d'une technique opératoire, par exemple percutanée, de traitement d'une pathologie osseuse, notamment du pied.

## Revendications

1. Instrument chirurgical (1) destiné à être manoeuvré à la manière d'un levier pour permettre de modifier le positionnement de corps osseux (2, 3) d'un patient l'un par rapport à l'autre, par exemple de son pied, ledit instrument chirurgical (1) comportant :
- un manche de préhension (5) s'étendant le long d'un premier axe longitudinal (A-A'),
- une tête de travail (8), comportant un corps (9) allongé s'étendant le long d'un deuxième axe longitudinal (B-B') entre une première extrémité (10) reliée au manche de préhension (5) et une deuxième extrémité (11) libre, ledit corps (9) présentant une surface supérieure (12) et une surface inférieure (13) reliant lesdites première (10) et deuxième (11) extrémités,
ledit instrument chirurgical (1) étant **caractérisé en ce que** la tête de travail (8) est pourvue d'une gorge (16) axiale ménagée dans ladite surface supérieure (12).

2. Instrument chirurgical (1) selon la revendication précédente, **caractérisé en ce que** ladite gorge (16) axiale s'étend entre une ouverture proximale (17) et une ouverture distale (18), lesdites ouvertures proximales (17) et distales (18) débouchant de la surface supérieure (12) du corps (9) de ladite tête de travail (8).

3. Instrument chirurgical (1) selon l'une quelconque des revendications précédentes, **caractérisé en ce que** ladite gorge (16) est formée par :
- un fond de gorge (19) s'étendant, à partir de l'ouverture proximale (17), le long de l'axe longitudinal (B-B') sur au moins une portion de la longueur de la tête de travail (8),
- deux parois (20) de gorge s'élevant à partir du fond (19) jusqu'à la surface supérieure (12), lesdites parois étant de préférence configurées de sorte à conférer à ladite gorge (16) un profil sensiblement conique.

4. Instrument chirurgical (1) selon l'une quelconque des revendications précédentes, **caractérisé en ce que** le corps (9) de la tête de travail (8) s'étend :
- latéralement entre deux bords latéraux (14), et
- transversalement entre lesdites surfaces supérieure (12) et inférieure (13),
lesdits bords latéraux (14) et lesdites surfaces supérieure (12) et inférieure (13) étant respectivement sensiblement parallèles sur au moins une portion de la longueur de la tête de travail (8), puis convergents vers l'extrémité (11) libre de cette dernière de sorte à former en cette extrémité (11) libre une pointe de travail (15).

5. Instrument chirurgical (1) selon l'une quelconque des revendications précédentes, **caractérisé en ce que** ladite tête de travail (8) est recourbée et **en ce que** le corps (9) de la tête de travail (8) s'étend de préférence sensiblement le long d'un plan d'extension proximal (Pp) sécant au premier axe longitudinal (A-A'), le corps (9) de la tête de travail (8) et le manche de préhension (5) étant reliés par une première zone de courbure (23).

6. Instrument chirurgical (1) selon la revendication précédente, **caractérisé en ce que** le corps (9) de la tête de travail (8) s'étend sensiblement le long d'un plan d'extension proximal (Pp), hormis sa pointe de travail (15) qui s'étend quant à elle sensiblement selon un plan d'extension distal (Pd) sécant au plan d'extension proximal (Pp), ladite pointe de travail (15) et ladite tête de travail (8) étant reliées par une deuxième zone de courbure (24).

7. Instrument chirurgical (1) selon les revendications 5 et 6, **caractérisé en ce que** lesdites ouverture proximale (17) et ouverture distale (18) de la gorge (16) débouchant de la surface supérieure (12) du corps (9) de ladite tête de travail (8) sont respectivement positionnées au niveau desdites première (23) et deuxième (24) zones de courbure.

8. Instrument chirurgical (1) selon l'une quelconque des revendications précédentes, **caractérisé en ce que** la tête de travail (8) est dotée de deux ailes longitudinales (25) faisant saillie du corps (9) allongé de ladite tête de travail (8), de sorte à en prolonger lesdites surfaces supérieure (12) et inférieure (13), lesdites ailes longitudinales (25) étant disposées symétriquement par rapport à un plan (Pg) d'extension de la gorge (16) et de façon radiale à l'axe longitudinal (B-B'), lesdites ailes longitudinales (25) étant de préférence positionnées à proximité de ladite première extrémité (10) de la tête de travail (8) et s'étendant de préférence longitudinalement sensiblement sur la moitié de la longueur de ladite tête de travail (8).

9. Instrument chirurgical (1) selon la revendication précédente, **caractérisé en ce que** lesdites ailes longitudinales (25) sont incurvées de sorte à conférer localement à ladite surface supérieure (12) une forme sensiblement concave et à ladite surface inférieure (13) une forme sensiblement convexe.

10. Instrument chirurgical (1) selon l'une quelconque des revendications précédentes, **caractérisée en ce que** les premier (A-A') et deuxième (B-B') axes sont confondus.

11. Instrument chirurgical (1) selon l'une quelconque des revendications précédentes, **caractérisé en ce qu'**il est à usage unique et, de préférence, réalisé en matériau polymère, par exemple un matériau polymère composite à base de polyarylamide (PAA), ce dernier étant optionnellement chargé.

12. Instrument chirurgical (1) selon l'une quelconque des revendications précédentes, **caractérisé en ce que** ledit manche de préhension (5) et ladite tête de travail (8) constituent une pièce monobloc d'un seul tenant.

13. Procédé de fabrication d'un instrument chirurgical (1) selon la revendication précédente, **caractérisé en ce qu'**il comprend une unique étape de moulage au cours de laquelle on réalise ledit instrument chirurgical (1) d'un seul tenant dans son intégralité.

14. Kit chirurgical (27) destiné à la mise en oeuvre d'une technique opératoire, préférentiellement percutanée, notamment dans le cadre du traitement d'une pathologie du pied d'un patient, comprenant un instrument chirurgical (1) selon l'une quelconque des revendications 1 à 12, et étant caractérisé en ce qui comprend également au moins un manche porte-lame de bistouri (28), une rugine (29) et une râpe (30), ledit kit chirurgical (27) étant de préférence à usage unique.

15. Kit chirurgical (27) selon la revendication précédente, **caractérisé en ce que** lesdits instrument chirurgical (1), manche porte-lame de bistouri (28), rugine (29) et râpe (30) sont tous réalisés en matériau polymère, par exemple un matériau polymère composite à base de polyarylamide (PAA), ce dernier étant optionnellement chargé.
